# EUROPEAN PATENT APPLICATION

(11) **EP 2 147 917 A1**
(43) Date of publication of application: **27.01.2010**
(21) Application number: 08752763.6
(22) Date of filing: 15.05.2008
(51) Int. Cl.: C07D 401/10

(54) **ONE-POT PRODUCTION PROCESS FOR CINNAMIDE DERIVATIVE**

(30) Priority: 16.05.2007 JP 2007130134; 16.05.2007 US 938349 P
(71) Applicant: Eisai R&D Management Co., Ltd., Tokyo 112-8088 (JP)
(72) Inventor: SHIMOMURA, Naoyuki, Tsukuba-shi Ibaraki 300-2635 (JP); YOSHIKAWA, Seiji, Kamisu-shi Ibaraki 314-0255 (JP); HISATAKE, Yoshihiko, Kamisu-shi Ibaraki 314-0255 (JP); IMAI, Akio, Kamisu-shi Ibaraki 314-0255 (JP)
(74) Representative: HOFFMANN EITLE
(86) International application number: PCT/JP2008/058902
(87) International publication number: WO 2008/140111

(57) **Abstract**

A compound represented by the formula (1) below or a pharmaceutically acceptable salt thereof is able to be produced in one pot by reacting a compound represented by the formula (2) below with 3-methoxy-4-(4-methyl-1H-imidazol-1-yl)benzaldehyde in the presence of a base and, if necessary, removing a protecting group. (In the formula (2), R₁ represents a 4-fluorophenyl group or the like; Q represents -CH(CH₃)-or the like; and n represents 1 or the like.) (In the formula (1), R represents a 4-fluorophenyl group or the like; Q and n are as defined above.)

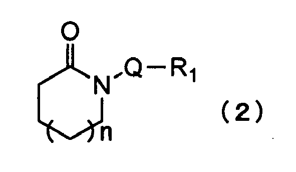

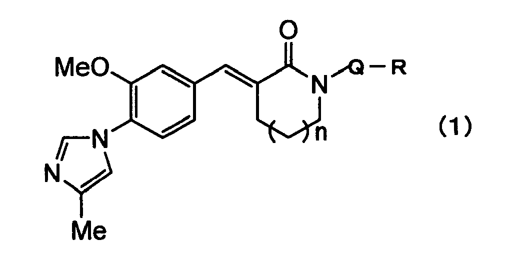

## Description

### TECHNICAL FIELD

The present invention relates to a method for preparing a compound represented by the following formula (1) or a pharmaceutically acceptable salt thereof: 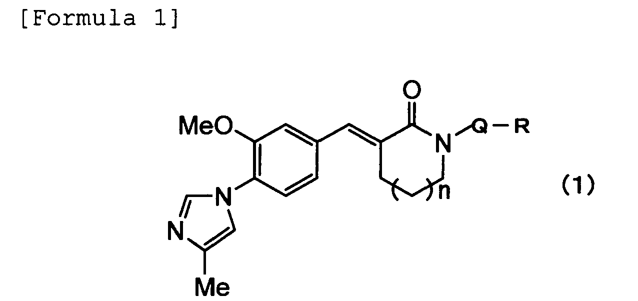 wherein R represents a 6- to 14-membered aromatic hydrocarbon ring group or a 5- to 14-membered aromatic heterocyclic group, both of which may have a substituent; Q represents a single bond or -CH(Y)-wherein Y represents a hydrogen atom or an alkyl group having 1 to 6 carbon atom(s); and n represents 0 to 2. In addition, the compound, particularly (3*E*)-1-[(1*S*)-1-(4-fluorophenyl)ethyl]-3-[3-methoxy-4-(4-methyl-1*H-*imidazol-1-yl)benzylidenelpiperidin-2-one has the effect of reducing β-amyloid production and is useful as an agent inhibiting the progression of or preventing a disease associated with amyloid beta, such as Alzheimer's disease.

### Background Art

Alzheimer's disease is a disease characterized by the degeneration or deciduation of nerve cells as well as the formation of senile plaques and the change of neurofibrils. The treatment of Alzheimer's disease is currently limited to symptomatic therapy using a symptom-improving agent exemplified by an acetylcholinesterase inhibitor, and no radical therapeutic agent inhibiting the progression of the disease has yet been developed. For creating a radical therapeutic agent for Alzheimer's disease, a method for controlling the pathogenesis of the disease state needs to be developed.
Aβ proteins, such as amyloid β40 and amyloid β42 (hereinafter referred to as Aβ40 and Aβ42), metabolites of amyloid precursor protein are thought to be significantly involved in the degeneration or deciduation of nerve cells, and further the onset of dementia symptoms (see, for example, Non-Patent Document 1 and Non-Patent Document 2). Accordingly, a compound reducing the production of Aβ40 and Aβ42 is expected as an agent inhibiting the progression of or preventing Alzheimer's disease.

The present inventors have found that a compound of the formula (1), particularly (3E)-1-[(1S)-1-(4-fluorophenyl)ethyl]-3-[3-methoxy-4-(4-methyl-1*H-*imidazol-1-yl)benzylidene]piperidin-2-one has the effect of reducing the production of Aβ40 and Aβ42 (See Patent Document 1). Furthermore, in Patent Document 1, a cyclic compound, wherein an amide moiety of a compound of the formula (1) is a piperidone ring, is disclosed in, for example, Examples 125 to 130 in which the cyclic compound is described as being able to be prepared by General Preparation Methods 3 to 5 described in Specification, pp. 106-114. In Patent Document 2, a preparation example of a cyclic compound wherein an amide moiety of a compound of the formula (1) is a piperidone ring, is described in Reference Example 1.
However, in Patent Document 1, examples are covered mainly in General Preparation Method 5, and a method for preparing a compound of the formula (1) in one-step reaction (one-pot reaction) by condensing a compound of the formula (2) according to the present invention with 3-methoxy-4-(4-methyl-1*H*-imidazol-1-yl)benzaldehyde is neither described nor suggested in either Patent Document 1 or Patent Document 2.
Non-Patent Document 1: Klein WL and 7 coauthors, Alzheimer's disease-affected brain: Presence of oligomeric Aβ ligands (ADDLs) suggests a molecular basis for reversible memory loss, Proceding National Academy of Science USA 2003, Sep 2; 100 (18), p. 10417-10422;
Non-Patent Document 2: Nitsch RM and 16 coauthors, Antibodies against β-amyloid slow cognitive decline in Alzheimer's disease, Neuron, 2003, May 22; 38, p. 547-554;
Patent Document 1: WO 2005/115990;
Patent Document 2: WO 2006/046575.

### Disclosure of Invention

### Problem to be solved by the Invention

An object of the present invention is to provide a novel method for preparing a compound of the formula (1), particularly (3*E*)-1-[(1*S*)-1-(4-fluorophenyl)ethyl]-3-[3-methoxy-4-(4-methyl-1*H-*imidazol-1-yl)benzylidenelpiperidin-2-one, which has the effect of reducing the production of Aβ, or a pharmaceutically acceptable salt thereof. Means for Solving the Problem

As a result of extensive studies to solve the problems, the present inventors have found that a high quality compound of the formula (1) can be prepared in a high yield in one-step reaction (one-pot reaction) by the following preparation method. This finding has led to the accomplishment of the present invention.
Specifically, the present invention relates to the following 1) to 5):
1) a method for preparing a compound represented by the following formula (1) or a pharmaceutically acceptable salt thereof: 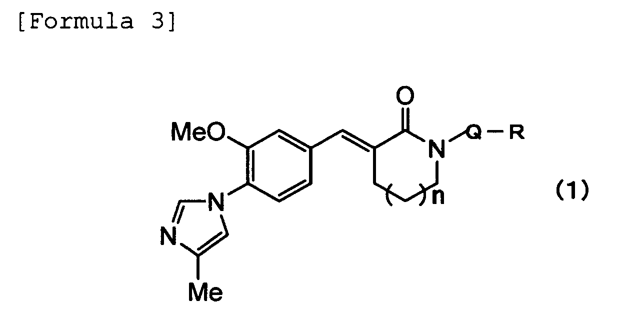 wherein R represents a 6- to 14-membered aromatic hydrocarbon ring group or a 5- to 14-membered aromatic heterocyclic group, both of which may have a substituent; Q represents a single bond or -CH(Y)-wherein Y represents a hydrogen atom or an alkyl group having 1 to 6 carbon atom(s); and n represents 0 to 2,
   comprises reacting a compound represented by the following formula (2): 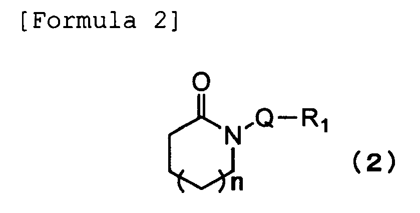 wherein R₁ represents a 6- to 14-membered aromatic hydrocarbon ring group or a 5- to 14-membered aromatic heterocyclic group, both of which may have an optionally protected substituent; and Q and n have the same meanings as described above,
   with 3-methoxy-4-(4-methyl-1*H*-imidazol-1-yl)benzaldehyde in the presence of a base and, if necessary, removing a protecting group;
2) the method according to the above 1) wherein the base is a C1-6 alkyllithium or a lithium amide compound;
3) the method according to the above 1) or 2) wherein the product obtained by a reaction using a C1-6 alkyllithium or a lithium amide compound as a base is further subjected, without isolation, to a reaction using an alkali metal C1-6 alkoxide, an amine compound, an alkali metal hydroxide or a carbonate compound as a base;
4) the method according to any one of the above 1) to 3) wherein the product obtained by a reaction using a C1-6 alkyllithium or a lithium amide compound as a base is further subjected, without isolation, to a reaction using an alkali metal C1-6 alkoxide or an amine compound; and
5) the method according to any one of the above 1) to 4) wherein Q in the formulas (1) and (2) is -CH(CH₃)-, and R in the formula (1) and R₁ in the formula (2) are a 4-fluorophenyl group.

Meanings of symbols, terms and the like used in the present specification will hereinafter be explained.

In the present specification, a structural formula of a compound may represent a certain isomer for convenience. However, the present invention includes all isomers such as geometric isomers which can be generated from the structure of a compound, optical isomers based on asymmetric carbon, stereoisomers and tautomers, and isomer mixtures. The present invention is not limited to the description of a chemical formula for convenience, and may include any one of isomers or mixtures thereof. Accordingly, the compound of the present invention may have an asymmetric carbon atom in the molecule, and exist as an optically active compound or a racemate, and the present invention includes both of the optically active compound and the racemate without limitations. Although crystal polymorphs of the compound may be present, the compound is not limited thereto as well and may be present as a single crystal form or a mixture thereof. The compound may be an anhydrate or a hydrate.

In compounds of the formulas (1) and (2) according to the present invention, the term "6- to 14-membered aromatic hydrocarbon ring group" and "5- to 14-membered aromatic heterocyclic group" have the following meanings.

The term "6- to 14-membered aromatic hydrocarbon ring group" refers to a monocylic, bicyclic or tricyclic aromatic hydrocarbon ring group having 6 to 14 carbons. Preferred examples of the group include monocyclic aromatic hydrocarbon ring groups such as a phenyl group; bicyclic aromatic hydrocarbon ring groups such as an indenyl group, a naphthyl group, an azulenyl group or a heptalenyl group; or tricyclic aromatic hydrocarbon ring groups such as a fluorenyl group, a phenalenyl group, a phenanthrenyl group or an anthracenyl group.

The term "5- to 14-membered aromatic heterocyclic group" refers to a monocylic, bicyclic or tricyclic aromatic heterocyclic group having 5 to 14 carbons. Preferred examples of the group include (1) nitrogen-containing aromatic heterocyclic groups such as a pyrrolyl group, a pyridyl group, a pyridazinyl group, a pyrimidinyl group, a pyrazinyl group, a pyrazolinyl group, an imidazolyl group, an indolyl group, an isoindolyl group, an indolidinyl group, a purinyl group, an indazolyl group, a quinolyl group, an isoquinolyl group, a quinolidinyl group, a phthalazinyl group, a naphthyridinyl group, a quinoxalinyl group, a quinazolinyl group, a cinnolinyl group, a pteridinyl group, an imidazotriazinyl group, a pyrazinopyridazinyl group, an acridinyl group, a phenanthridinyl group, a carbazolyl group, a perimidinyl group and a phenanthrolinyl group; (2) sulfur-containing aromatic heterocyclic groups such as a thienyl group and a benzothienyl group; (3) oxygen-containing aromatic heterocyclic groups such as a furyl group, a pyranyl group, a cyclopentapyranyl group, a benzofuranyl group and an isobenzofuranyl group; and (4) aromatic heterocyclic groups containing two or more hetero atoms selected from the group consisting of a nitrogen atom, a sulfur atom and an oxygen atom such as a thiazolyl group, an isothiazolyl group, a benzothiazolinyl group, a benzothiadiazolyl group, a phenothiazinyl group, an isoxazolyl group, a furazanyl group, a phenoxazinyl group, a pyrazoloxazolyl group, an imidazothiazolyl group, a thienofuryl group, a furopyrrolyl group and a pyridooxazinyl group.

In "6- to 14-membered aromatic hydrocarbon ring group which may have an optionally protected substituent", "5- to 14-membered aromatic heterocyclic group which may have an optionally protected substituent", "6- to 14-membered aromatic hydrocarbon ring group which may have a substituent", and "5- to 14-membered aromatic heterocyclic group which may have a substituent", the term "substituent" refers to a substituent selected from Substituent Group A1. The aromatic hydrocarbon ring group and the aromatic heterocyclic group may have 1 to 3 substituent(s), which may be the same or different.
Substituent Group A1 refers to (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group that may be substituted with 1 to 3 substituent(s) selected from Substituent Group A2, (19) a C1-6 alkoxy group that may be substituted with 1 to 3 substituent(s) selected from Substituent Group A2, (20) a C1-6 alkylthio group that may be substituted with 1 to 3 substituent(s) selected from Substituent Group A2, (21) an amino group that may be substituted with 1 or 2 substituent(s) selected from Substituent Group A2, (22) a carbamoyl group that may be substituted with 1 or 2 substituent(s) selected from Substituent Group A2, (23) a 6- to 14-membered aromatic hydrocarbon ring group that may be substituted with 1 to 5 substituent(s) selected from Substituent Group A2, (24) a 5- to 14-membered aromatic heterocyclic group that may be substituted with 1 to 3 substituent(s) selected from Substituent Group A2, (25) a 6- to 14-membered non-aromatic hydrocarbon ring group that may be substituted with 1 to 3 substituent(s) selected from Substituent Group A2, (26) a 5- to 14-membered non-aromatic heterocyclic group that may be substituted with 1 to 3 substituent(s) selected from Substituent Group A2, (27) a C2-6 alkenyloxy group, (28) a C2-6 alkynyloxy group, (29) a C3-8 cycloalkylsulfinyl group, (30) a C3-8 cycloalkylsulfonyl group, (31) -X-A wherein X represents an imino group, -O- or -S-, and A represents a 6- to 14-membered aromatic hydrocarbon ring group or a 5- to 14-membered aromatic heterocyclic group, both of which may be substituted with 1 to 3 substituent(s) selected from Substituent Group A2, (32) -CO-A wherein A has the same meaning as described above and (33) =CH-A wherein A has the same meaning as described above. Substituent Group A2 used herein refers to (1) a hydrogen atom, (2) a halogen atom, (3) a hydroxyl group, (4) a cyano group, (5) a nitro group, (6) a C1-6 alkyl group, (7) a C3-8 cycloalkyl group, (8) a C2-6 alkenyl group, (9) a C2-6 alkynyl group, (10) a C1-6 alkoxy group, (11) a C3-8 cycloalkoxy group (12) a C3-8 cycloalkylthio group, (13) a formyl group, (14) a C1-6 alkylcarbonyl group, (15) a C1-6 alkylthio group, (16) a C1-6 alkylsulfinyl group, (17) a C1-6 alkylsulfonyl group, (18) a hydroxyimino group and (19) a C1-6 alkoxyimino group.

In the above Substituent Group(s) A1 and/or A2, the term "halogen atom" refers to a fluorine atom, a chlorine atom, a bromine atom or an iodine atom, and preferably, a fluorine atom, a chlorine atom or a bromine atom.

The term "C1-6 alkyl group" refers to an alkyl group having 1 to 6 carbon atom(s). Preferred examples of the group include linear or branched alkyl groups such as a methyl group, an ethyl group, a *n-*propyl group, an *i*-propyl group, a *n*-butyl group, an *i-*butyl group, a *tert-*butyl group, a *n*-pentyl group, an *i*-pentyl group, a neopentyl group, a *n*-hexyl group, a 1-methylpropyl group, a 1,2-dimethylpropyl group, a 1-ethylpropyl group, a 1-methyl-2-ethylpropyl group, a 1-ethyl-2-methylpropyl group, a 1,1,2-trimethylpropyl group, a 1-methylbutyl group, a 2-methylbutyl group, a 1,1-dimethylbutyl group, a 2,2-dimethylbutyl group, a 2-ethylbutyl group, a 1,3-dimethylbutyl group, a 2-methylpentyl group or a 3-methylpentyl group.

The term "C1-6 alkoxy group" refers to an alkyl group having 1 to 6 carbon atom(s) in which one hydrogen atom is replaced by an oxygen atom. Preferred examples of the group include a methoxy group, an ethoxy group, a *n*-propoxy group, an *i*-propoxy group, a *n*-butoxy group, an *i*-butoxy group, a *sec*-butoxy group, a *tert-*butoxy group, a *n*-pentyloxy group, an *i-*pentyloxy group, a *sec*-pentyloxy group, a *tert-*pentyloxy group, a *n*-hexyloxy group, an *i*-hexyloxy group, a 1,2-dimethylpropoxy group, a 2-ethylpropoxy group, a 1-methyl-2-ethylpropoxy group, a 1-ethyl-2-methylpropoxy group, a 1,1,2-trimethylpropoxy group, a 1,1-dimethylbutoxy group, a 2,2-dimethylbutoxy group, a 2-ethylbutoxy group, a 1,3-dimethylbutoxy group, a 2-methylpentyloxy group or a 3-methylpentyloxy group.

The term "C1-6 alkylsulfonyl group" refers to an alkyl group having 1 to 6 carbon atom(s) in which one hydrogen atom is replaced by a sulfonyl group. Preferred examples of the group include a methanesulfonyl group or an ethanesulfonyl group.

The term "amino group that may be substituted with a C1-6 alkyl group" refers to an amino group that may be substituted with an alkyl group having 1 to 6 carbon atom(s). Preferred examples of the group include an amino group, a methylamino group, an ethylamino group, a propylamino group or a dimethylamino group.

The term "C2-6 alkenyl group" refers to an alkenyl group having 2 to 6 carbon atoms. Preferred examples of the group include linear or branched alkenyl groups such as a vinyl group, an allyl group, a 1-propenyl group, an isopropenyl group, a 1-buten-1-yl group, a 1-buten-2-yl group, a 1-buten-3-yl group, a 2-buten-1-yl group or a 2-buten-2-yl group.

The term "C2-6 alkynyl group" refers to an alkynyl group having 2 to 6 carbon atoms. Preferred examples of the group include linear or branched alkynyl groups such as an ethynyl group, a 1-propynyl group, a 2-propynyl group, a butynyl group, a pentynyl group or a hexynyl group.

The term "C3-8 cycloalkyl group" refers to a cyclic alkyl group having 3 to 8 carbon atoms. Preferred examples of the group include a cyclopropyl group, a cyclobutyl group, a cyclopentyl group, a cyclohexyl group, a cycloheptyl group or a cyclooctyl group.

The term "C1-6 alkylthio group" refers to an alkyl group having 1 to 6 carbon atom(s) in which one hydrogen atom is replaced by a sulfur atom. Preferred examples of the group include a methylthio group, an ethylthio group, a *n*-propylthio group, an *i*-propylthio group, a *n*-butylthio group, an *i*-butylthio group, a tert-butylthio group, a *n*-pentylthio group, an *i-*penthylthio group, a neopenthylthio group, a n-hexylthio group or a 1-methylpropylthio group.

The term "C1-6 alkylsulfinyl group" refers to an alkyl group having 1 to 6 carbon atom(s) in which one hydrogen atom is replaced by a sulfinyl group. Preferred examples of the group include a methanesulfinyl group or an ethanesulfinyl group.

The term "C1-6 alkylcarbonyl group" refers to an alkyl group having 1 to 6 carbon atom(s) in which one hydrogen atom is replaced by a carbonyl group. Preferred examples of the group include an acetyl group, a propionyl group or a butyryl group.

The term "C3-8 cycloalkoxy group" refers to a cyclic alkyl group having 3 to 8 carbon atoms in which one hydrogen atom is replaced by an oxygen atom. Preferred examples of the group include a cyclopropoxy group, a cyclobutoxy group, a cyclopentyloxy group, a cyclohexyloxy group, a cycloheptyloxy group or a cyclooctyloxy group.

The term "C3-8 cycloalkylthio group" refers to a cyclic alkyl group having 3 to 8 carbon atoms in which one hydrogen atom is replaced by a sulfur atom. Preferred examples of the group include a cyclopropylthio group, a cyclobutylthio group, a cyclopentylthio group, a cyclohexylthio group, a cycloheptylthio group or a cyclooctylthio group.

The term "C1-6 alkoxyimino group" refers to a group in which a hydrogen atom in an imino group is replaced by a C1-6 alkoxy group. Preferred examples of the group include a methoxyimino group or an ethoxyimino group.

The term "C2-6 alkenyloxy group" refers to an alkenyl group having 2 to 6 carbon atoms in which one hydrogen atom is replaced by an oxygen atom. Preferred examples of the group include linear or branched alkenyloxy groups such as a vinyloxy group, an allyloxy group, a 1-propenyloxy group, an isopropenyloxy group, a 1-buten-1-yloxy group, a 1-buten-2-yloxy group, a 1-buten-3-yloxy group, a 2-buten-1-yloxy group or a 2-buten-2-yloxy group.

The term "C2-6 alkynyloxy group" refers to an alkynyl group having 2 to 6 carbon atoms in which one hydrogen atom is replaced by an oxygen atom. Preferred examples of the group include linear or branched alkynyloxy groups such as an ethynyloxy group, a 1-propynyloxy group, a 2-propynyloxy group, a butynyloxy group, a pentynyloxy group or a hexynyloxy group.

The term "C3-8 cycloalkylsulfinyl group" refers to a cyclic alkyl group having 3 to 8 carbon atoms in which one hydrogen atom is replaced by a sulfinyl group. Preferred examples of the group include a cyclopropanesulfinyl group, a cyclobutanesulfinyl group, a cyclopentanesulfinyl group, a cyclohexanesulfinyl group, a cycloheptanesulfinyl group or a cyclooctanesulfinyl group.

The term "C3-8 cycloalkylsulfonyl group" refers to a cyclic alkyl group having 3 to 8 carbon atoms in which one hydrogen atom is replaced by a sulfonyl group. Preferred examples of the group include a cyclopropanesulfonyl group, a cyclobutanesulfonyl group, a cyclopentanesulfonyl group, a cyclohexanesulfonyl group, a cycloheptanesulfonyl group or a cyclooctanesulfonyl group.

The term "6- to 14-membered non-aromatic hydrocarbon ring group" refers to a cyclic aliphatic hydrocarbon group having 6 to 14 carbon atoms which constitute the ring. Preferred examples of the group include an aliphatic hydrocarbon group such as a cyclopropyl group, a cyclobutyl group, a cyclopentyl group, a cyclohexyl group, a cycloheptyl group, a cyclooctyl group, a spiro[3,4]octanyl group, a decanyl group, an indanyl group, a 1-acenaphtenyl group, a cyclopentacyclooctenyl group, a benzocyclooctenyl group, an indenyl group, a tetrahydronaphthyl group, a 6,7,8,9-tetrahydro-5H-benzocycloheptenyl group or a 1,4-dihydronaphthalenyl group.

The term "5- to 14-membered non-aromatic heterocyclic group" means not only a 5- to 14-membered non-aromatic heteromonocyclic group but also a saturated heterocyclic group condensed with aromatic hydrocarbon ring groups, or a saturated hydrocarbon ring group or a saturated heterocyclic group condensed with aromatic heterocyclic groups, which (1) has 5 to 14 ring-constituting atoms, (2) contains 1 to 5 heteroatom(s) such as nitrogen, -O- or -S- in the ring-constituting atoms, and (3) may contain one or more carbonyl groups, double bonds or triple bonds in the ring. Specific examples of the 5- to 14-membered non-aromatic heterocyclic group include an azetidinyl group, a pyrrolidinyl group, a piperidinyl group, an azepanyl group, an azocanyl group, a tetrahydrofuranyl group, a tetrahydropyranyl group, a morpholinyl group, a thiomorpholinyl group, a piperazinyl group, a thiazolidinyl group, a dioxanyl group, an imidazolinyl group, a thiazolinyl group, a 1,2-benzopyranyl group, an isochromanyl group, a chromanyl group, an indolinyl group, an isoindolinyl group, an azaindanyl group, an azatetrahydronaphthyl group, an azachromanyl group, a tetrahydrobenzofuranyl group, a tetrahydrobenzothienyl group, a 2,3,4,5-tetrahydrobenzo[b]thienyl group, a 3,4-dihydro-2H-benzo[b][1,4]dioxepinyl group, an indan-1-onyl group, a 6,7-dihydro-5H-cyclopentapyrazinyl group, a 6,7-dihydro-5H-[1]pyridinyl group, a 6,7-dihydro-5H-[1]pyridinyl group, a 5,6-dihydro-4H-cyclopenta[b]thienyl group, a 4,5,6,7-tetrahydro-benzo[b]thienyl group, a 3,4-dihydro-2H-naphtal-1-onyl group, a 2,3-dihydro-isoindol-1-onyl group, a 3,4-dihydro-2H-isoquinolin-1-onyl group or a 3,4-dihydro-2H-benzo[1,4]oxapinyl group.]

In the above Substituent Group A1, the "6- to 14-membered aromatic hydrocarbon ring group" and "5- to 14-membered aromatic heterocyclic group" have the same meanings as the above "6- to 14-membered aromatic hydrocarbon ring group" and "5- to 14-membered aromatic heterocyclic group" in compounds of the formulas (1) and (2) according to the present invention.

In a compound of the formula (2) according to the present invention, examples of a protecting group for a substituent in "6- to 14-membered aromatic hydrocarbon ring group or 5- to 14-membered aromatic heterocyclic group, both of which may have an optionally protected substituent" include commonly used protecting groups for a hydroxyl group, a carboxyl group, a carbonyl group or an amino group. The protecting group includes, but is not limited to, one described in Protective groups In Organic Synthesis Second Edition by T.W. Greene and P.G. M. Wuts John Wiley & Sons, Inc.

Examples of a protecting group for a hydroxyl group include a methoxymethyl group, a methylthiomethyl group, a tetrahydrofuranyl group, a 1-ethoxyethyl group, a *tert-*butyldimethylsilyl group, a benzyl group, a *tert-*butyl group, an allyl group or a triphenylmethyl group.
Examples of a protecting group for a carboxyl group include a methyl group, an ethyl group, a 2,2,2-trichloroethyl group, an ethoxycarbonyl group, a methoxycarbonyl group, a benzyl group, an o-nitrobenzyl group, a *p*-nitrobenzyl group, a β-*p-*toluenesulfonylethyl group, a *p*-methoxybenzyl group or a benzyloxycarbonyl group.

Examples of a protecting group for a carbonyl group include a 1,3-dioxanyl group, a 5-methylene-1,3-dioxanyl group or a 5,5-dibromo-1,3-dioxanyl group.
Examples of a protecting group for an amino group include an N-formyl group, an N-acetyl group, an N-chloroacetyl group, an N-benzoyl group, a *tert*-butyl group, an N-phthalimide group, a diphenylmethyl group or a benzyl group, and one or two protecting group(s) described above can be introduced into an amino group accordingly.

In a compound of the formula (2) according to the present invention, examples of "an alkyl group having 1 to 6 carbon atom(s) " for Y in the formula - CH(Y)- of Q are similar to those described in the above "C1-C6 alkyl group".

Preparation methods according to the present invention will hereinafter be described in detail.

A compound represented by the following formula (1) or a pharmaceutically acceptable salt thereof: 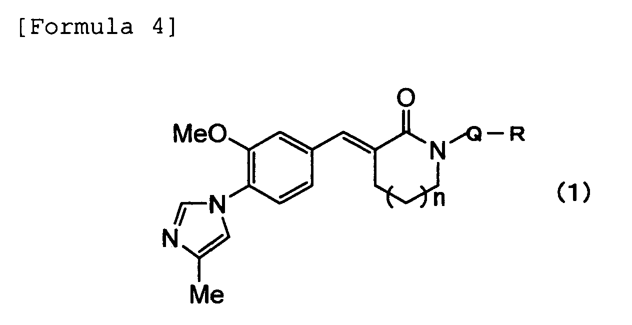 wherein R represents a 6- to 14-membered aromatic hydrocarbon ring group or a 5- to 14-membered aromatic heterocyclic group, both of which may have a substituent; Q represents a single bond or -CH(Y)-wherein Y represents a hydrogen atom or an alkyl group having 1 to 6 carbon atom(s); and n represents 0 to 2,
can be prepared by reacting a compound represented by the following formula (2): 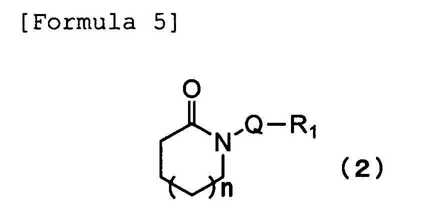 wherein R₁ represents a 6- to 14-membered aromatic hydrocarbon ring group or a 5- to 14-membered aromatic heterocyclic group, both of which may have an optionally protected substituent; and Q and n have the same meanings as described above,
with 3-methoxy-4-(4-methyl-1*H*-imidazol-1-yl)benzaldehyde in the presence of a base and, if necessary, removing a protecting group therefrom.

The reaction is performed by reacting a compound of the formula (2) with 3-methoxy-4-(4-methyl-1*H*-imidazol-1-yl)benzaldehyde in a solvent, in the presence of a base, and then removing a protecting group when contained in the resulting compound. In the reaction, the compound of the formula (2) and 3-methoxy-4-(4-methyl-1*H*-imidazol-1-yl)benzaldehyde are used theoretically in equimolar amounts. For smooth reaction, however, the amount can be increased or decreased accordingly to control the reaction. The compound of the formula (2) can be used preferably, for example, in a 1.0-fold to 1.5-fold molar amount, and more preferably, for example, a 1.0-fold to 1.3-fold molar amount relative to 3-methoxy-4-(4-methyl-1*H-*imidazol-1-yl)benzaldehyde.
The solvent used in the reaction may be either an organic solvent or a water-containing solvent, but preferably an organic solvent. There is no particular restriction on the solvent, but the solvent not easily reacting with raw materials is preferred. Preferred examples of the solvent include a solvent not affecting the reaction such as 1,4-dioxane, tetrahydrofuran, 1,2-dimethoxyethane, methyl *tert-*butyl ether, cyclopentyl methyl ether, diethylether, diisopropylether, dibutylether, dicyclopentylether, benzene, toluene, xylene, *n*-hexane, *c*-hexane, *n*-heptane or tetrahydropyran; or a mixed solvent thereof. More preferred examples thereof include a mixed solvent of tetrahydrofuran and toluene.
Preferred examples of the base used in the reaction include C1-6 alkyllithium such as *n-*butyllithium, *sec*-butyllithium or *tert*-butyllithium; or a lithium amide compound such as lithium diisopropylamide or 2,2,6,6-tetramethylpiperidine lithium amide. More preferred examples thereof include lithium diisopropylamide.

The amount of the base used in the reaction may be increased or decreased accordingly, but the amount thereof is preferably, for example, a 1.0-fold to 3.0-fold molar amount, and more preferably, for example, a 1.1-fold to 1.4-fold molar amount relative to 3-methoxy-4-(4-methyl-1*H*-imidazol-1-yl)benzaldehyde.
The reaction temperature generally varies depending on the starting material, the solvent and the reagent used in the reaction, and can be changed accordingly. The reaction temperature is preferably, for example, from -78°C to 10°C, and more preferably, for example, from -20°C to 10°C.
The reaction time generally varies depending on the starting material, the solvent and the reagent used in the reaction as well as the reaction temperature and the progress of the reaction, and can be increased or decreased accordingly. After addition of the base, the reaction is generally completed in preferably, for example, 10 minutes to 2 hours, and more preferably, for example, around 30 minutes at the above reaction temperature.

Preferably, in the preparation method of the present invention, a product obtained by reacting the compound of the formula (2) with 3-methoxy-4-(4-methyl-1*H*-imidazol-1-yl)benzaldehyde in the presence of the base is further subjected, without isolation, to the reaction in the presence of the base. Preferred examples of the base used herein include alkali metal C1-6 alkoxide such as sodium ethoxide, sodium methoxide, sodium *tert-*butoxide and potassium *tert-*butoxide; amine compounds such as triethylamine, pyridine and diisopropylethylamine; alkali metal hydroxide such as lithium hydroxide, sodium hydroxide or potassium hydroxide; a carbonate compound such as lithium carbonate, sodium carbonate, potassium carbonate, cesium carbonate, sodium hydrogen carbonate or potassium hydrogen carbonate. More preferred examples thereof include alkali metal C1-C6 alkoxide such as sodium ethoxide, sodium methoxide, sodium *tert-*butoxide or potassium *tert-*butoxide; or amine compounds such as triethylamine, pyridine or diisopropylethylamine.

The amounts of the base used in the reaction may be increased or decreased accordingly, but the amount thereof is preferably, for example, a 1.0-fold to 10.0-fold molar amount, and more preferably, for example, a 1.0-fold to 4.0-fold molar amount relative to 3-methoxy-4-(4-methyl-1*H*-imidazol-1-yl)benzaldehyde. The reaction temperature generally varies depending on the starting material, the solvent and the reagent used in the reaction, and can be changed accordingly. The reaction temperature is preferably, for example, from - 10°C to 50°C, and more preferably, for example, from 0°C to 30°C. In general, the reaction time is preferably, for example, 0.5 to 10 hour(s), and more preferably, for example, 0.5 to 1 hour, at the above reaction temperature.

In the preparation method of the present invention, the method of removing a protecting group varies depending on the type of the protecting group and the stability of the compound, but it can be performed by, for example, a solvolysis with acids or bases, a chemical reduction with complex metal hydrides, or a catalytic reduction with palladium carbon catalysts or Raney nickel catalysts, according to the method described in the literature (see T.W. Greene, "Protective groups in Organic Synthesis" John Wiley & Sons, 1981) or the method in conformity thereto.
After the reaction, objects for each reaction are collected from the reaction mixture according to an ordinary method. For example, when an insoluble matter exists, it is filtered off accordingly, and then the solvent is distilled off under reduced pressure to obtain the target compound. Alternatively, the reaction mixture is diluted with an organic solvent such as ethyl acetate and washed with water, and then the organic layer is dried over anhydrous magnesium sulfate and the solvent is distilled off to obtain the target compound. The resulting compound can be further purified by an ordinary method such as column chromatography, thin layer chromatography or high pressure liquid chromatography, if necessary.

Furthermore, a compound of the formula (1) may be prepared as a pharmaceutically acceptable salt by an ordinary method. The preparation method can be performed in combination with, for example, methods generally used in the field of synthetic organic chemistry accordingly. There can be mentioned particularly a method in which a solution containing a free compound of the formula (1) according to the present invention is subjected to neutralization titration with a predetermined acidic solution or an alkaline solution.
The term "pharmaceutically acceptable salt" used herein is not particularly limited as long as it is formed with a compound of the formula (1) as a medicament for the prevention and treatment of diseases caused by Aβ, as a pharmacologically acceptable salt. Preferred examples of the salt include hydrohalide (for example, hydrofluoride, hydrochloride, hydrobromide and hydroiodide), inorganic acid salt (for example, sulfate, nitrate, perchlorate, phosphate, carbonate or bicarbonate), organic carboxylate (for example, acetate, oxalate, maleate, tartrate, fumarate and citrate), organic sulfonate (for example, methanesulfonate, trifluoromethanesulfonate, ethanesulfonate, benzenesulfonate, toluenesulfonate or camphorsulfonate), amino acid salt (for example, aspartate or glutamate), quaternary amine salt, alkali metal salt (for example, sodium salt or potassium salt) or alkaline earth metal salt (for example, magnesium salt or calcium salt).

A compound of the formula (2) as a raw material in the preparation method of the present invention can be prepared by reacting a compound of the following formula (3):

[Formula 6] **R₁-Q - NH₂** **(3)**

wherein R₁ and Q have the same meanings as described above,
with a compound of the following formula (4) in a solvent in the presence of a base:

[Formula 7] **X₁-(CH₂)₂-(CH₂n-CH₂-CO-X₂** (4)

wherein X₁ and X₂ represent a halogen atom, and n has the same meaning as described above.

This process can be performed according to an ordinary method described in Shin Jikken Kagaku Koza (New Courses in Experimental Chemistry), vol. 14, Yuki Kagobutsu No Gosei To Hannou (Synthesis and Reaction of Organic Compounds) [II], pp. 1134-1188, for example. Specifically, a compound of the formula (2) as a raw material can be prepared by reacting a compound of the formula (3) with a compound of the formula (4) in a water-containing solvent in the presence of a base and a phase transfer catalyst. Alternatively, a compound of the formula (3) is reacted with a compound of the formula (4) to obtain an open chain amide, which is then subjected to a cyclization reaction in the presence of a base so as to prepare the compound of the formula (2).

Firstly, a method of preparing the raw material in a water-containing solvent in the presence of a base or a phase transfer catalyst will be described in detail.

There is no particular restriction on the solvent used in the reaction as long as it dissolves the starting material to some extent and does not inhibit the reaction, which may be any of an organic solvent, a water-containing solvent and a biphasic mixed solvent of an organic solvent and water, but preferred examples of the solvent include a mixture of water and solvent such as benzene, toluene, xylene, isopropanol, tetrahydrofuran, *tert-*butyl methyl ether, cyclopentyl methyl ether, acetonitrile, *N,N-*dimethylformamide, 1,4-dioxane, 1,2-dimethoxyethane, 1-methyl-2-pyrrolidone or 1,3-dimethyl-2-imidazolydinone, and more preferably a mixture of water and solvent such as toluene or *tert-*butyl methyl ether.

The amounts of a compound of the formula (4) may be increased or decreased accordingly, but the amount thereof is preferably, for example, a 1-fold to 3-fold molar amount, and more preferably, for example, a 1.0-fold to 1.3-fold molar amount relative to a compound of the formula (3).

Preferred examples of the base used in the reaction include lithium hydroxide, sodium hydroxide, potassium hydroxide, lithium carbonate, sodium carbonate, potassium carbonate, cesium carbonate, sodium hydrogen carbonate or potassium hydrogen carbonate. More preferred examples thereof include sodium hydroxide, potassium hydroxide, sodium carbonate or potassium carbonate.
The amounts of the base used in the reaction may be increased or decreased accordingly, but the amount thereof is preferably, for example, a 1.0-fold to 50.0-fold molar amount, and more preferably, for example, a 1.0-fold to 30.0-fold molar amount relative to a compound of the formula (3).

Preferred examples of the phase transfer catalyst used in the reaction include benzyltriethylammonium chloride, benzyltriethylammonium bromide, tetra-*n*-butylammonium fluoride, tetra-*n-*butylammonium chloride or tetra-*n*-butylammonium bromide. More preferred examples thereof include benzyltriethylammonium chloride.
The amounts of the phase transfer catalyst used in the reaction may be increased or decreased accordingly, but the amount thereof is preferably, for example, a 0.05-fold to 0.5-fold molar amount, and more preferably, for example, a 0.05-fold to 0.15-fold molar amount relative to a compound of the formula (3).

The reaction temperature generally varies depending on the starting material, the solvent and the reagent used in the reaction, and can be changed accordingly. The reaction temperature is preferably, for example, from -5°C to 50°C, and more preferably, for example, from 0°C to 30°C.
The reaction time can be generally increased or decreased accordingly depending on the starting material, the solvent and the reagent used in the reaction as well as the reaction temperature and the progress of the reaction. The reaction time is preferably, for example, 0.5 to 200 hour(s), and more preferably, for example, around 96 hours at the above reaction temperature.

Secondly, a production process in which an open chain amide obtained by reacting a compound of the formula (3) with a compound of the formula (4) is further subjected to a cyclization reaction in the presence of a base will now be described in detail.

There is no particular restriction on the solvent used in the reaction to obtain an open chain amide as long as it dissolves the starting material to some extent and does not inhibit the reaction, but preferred examples of the solvent include a mixture of water and solvent such as benzene, toluene, xylene, isopropanol, tetrahydrofuran, *tert-*butyl methyl ether, cyclopentyl methyl ether, acetonitrile, *N,N-*dimethylformamide, 1,4-dioxane, 1,2-dimethoxyethane, 1-methyl-2-pyrrolidone or 1,3-dimethyl-2-imidazolydinone. More preferred examples thereof include a mixture of water and solvent such as toluene, cyclopentyl methyl ether or *tert-*butyl methyl ether.

Preferred examples of the base used in the reaction to obtain an open chain amide include lithium hydroxide, sodium hydroxide, potassium hydroxide, lithium carbonate, sodium carbonate, potassium carbonate, cesium carbonate, sodium hydrogen carbonate or potassium hydrogen carbonate. More preferred examples thereof include sodium hydroxide, potassium hydroxide, sodium carbonate or potassium carbonate.

The amount of the base used in the reaction to obtain an open chain amide may be increased or decreased accordingly, but the amount thereof is preferably, for example, a 1.0-fold to 10.0-fold molar amount, and more preferably, for example, a 1.0-fold to 3.0-fold molar amount relative to a compound of the formula (3).

The reaction temperature for the open chain amide generally varies depending on the starting material, the solvent and the reagent used in the reaction and can be changed accordingly. The reaction temperature is preferably, for example, from -5°C to 50°C, and more preferably, for example, from 0°C to 30°C.

The reaction time for the open chain amide can be generally increased or decreased accordingly depending on the starting material, the solvent and the reagent used in the reaction as well as the reaction temperature and the progress of the reaction. The reaction time is preferably, for example, 0.5 to 200 hour(s), and more preferably, for example, about 1 to 2 hour(s) at the above reaction temperature.

There is no particular restriction on the solvent used in the reaction to cyclize an open chain amide so as to obtain a compound of the formula (2) as long as it dissolves the starting material to some extent and does not inhibit the reaction, but preferred examples of the solvent include a solvent such as benzene, toluene, xylene, isopropanol, tetrahydrofuran, *tert-*butyl methyl ether, cyclopentyl methyl ether, acetonitrile, *N,N*-dimethylformamide, 1,4-dioxane, 1,2-dimethoxyethane, 1-methyl-2-pyrrolidone or 1,3-dimethyl-2-imidazolydinone; a mixed solvent thereof; or a mixture of water and the solvent or the mixed solvent. More preferred examples thereof include a solvent such as tetrahydrofuran, toluene, cyclopentyl methyl ether or *tert-*butyl methyl ether; a mixed solvent thereof; or a mixture of water and the solvent or the mixed solvent.

Preferred examples of the base used in the reaction to cyclize an open chain amide so as to obtain a compound of the formula (2) include sodium ethoxide, sodium methoxide, sodium *tert-*butoxide, potassium *tert-*butoxide, triethylamine, pyridine, diisopropylethylamine, lithium hydroxide, sodium hydroxide, potassium hydroxide, lithium carbonate, sodium carbonate, potassium carbonate, cesium carbonate, sodium hydrogen carbonate or potassium hydrogen carbonate. More preferred examples thereof include sodium ethoxide, sodium methoxide or potassium *tert-*butoxide.

The amount of the base used in the reaction to cyclize an open chain amide so as to obtain a compound of the formula (2) can be increased or decreased accordingly, but the amount thereof is preferably, for example, a 1.0-fold to 10.0-fold molar amount, and more preferably, for example, a 1.0-fold to 3.0-fold molar amount relative to a compound of the formula (3).

The reaction temperature to cyclize an open chain amide so as to obtain a compound of the formula (2) generally varies depending on the starting material, the solvent and the reagent used in the reaction, and can be changed accordingly. The reaction temperature is preferably, for example, from -5°C to 50°C, and more preferably, for example, from 0°C to 30°C.

The reaction time to cyclize an open chain amide so as to obtain a compound of the formula (2) can be generally increased or decreased accordingly depending on the starting material, the solvent and the reagent used in the reaction, the reaction temperature as well as the progress of the reaction. The reaction time is preferably, for example, 0.5 to 10 hour(s), and more preferably, for example, 0.5 to 1 hour at the above reaction temperature.

Known compounds, commercially available compounds, or compounds which may be easily prepared from commercially available compounds by the technique known in the art can be used as compounds of the formulas (3) and (4).

3-Methoxy-4-(4-methyl-1*H*-imidazol-1-yl)benzaldehyde as a raw material in the preparation method of the present invention is known and described in Specification p. 126 [0145], Patent Document 1, WO 2005/115990.

### Best Mode for Carrying out the Invention

The present invention will now be described in more detail with reference to the following Production Examples and Examples. However, the Examples are provided only for illustration purposes. The preparation method according to the present invention is not limited to the following specific examples in any case. Those skilled in the art can fully implement the present invention by making various modifications to not only the following examples but also various aspects of the present specification, which are within the scope of the claims of the present specification.

### Production Example 1

### Preparation of 1-[1-(4-fluorophenyl)ethyl]piperidin-2-one

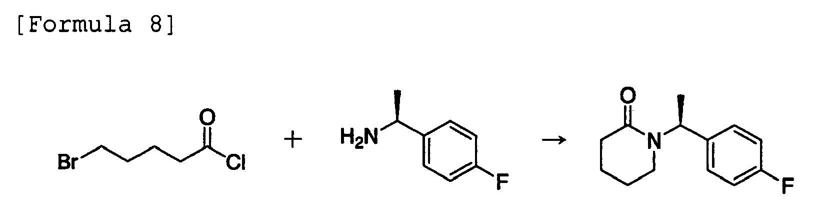

A solution of 5-bromovaleryl chloride (1.0 ml) in toluene (2 ml) was added dropwise to a bilayer mixture of a vigorously-stirred solution of 1.0 g of (*S*)-1-(4-fluorophenyl)ethylamine (7.19 mmol) in 5 ml of toluene and an aqueous 50% sodium hydroxide solution (7 ml) under ice-cooling over 13 minutes. The reaction mixture was stirred at the same temperature for 15 minutes, and then benzyltriethylammonium chloride (164 mg) was added thereto. The reaction mixture was further stirred at room temperature for 4 days. To the reaction solution was added 30 ml of ice water, and the solution was separated. The aqueous layer was then reextracted with toluene (7 ml). The combined organic layers were washed sequentially with water, 1 N hydrochloride, water, a saturated aqueous sodium hydrogen carbonate solution and a saturated saline solution, and dried over anhydrous magnesium sulfate. Thereafter, the solvent was distilled off under reduced pressure to obtain 1.38 g (yield: 87%) of the title compound as a colorless oily substance.
¹H-NMR (600 MHz, CDCl₃) δ (ppm): 1.49 (d, *J* = 7 Hz, 3H), 1.60 (m, 1H), 1.74 (overlapped, 1H), 1.76 (overlapped, 2H), 2.48 (m, 2H), 2.77 (m, 1H), 3.10 (m, 1H), 6.13 (q, *J* = 7 Hz, 1H), 7.01 (dd, *J* = 9, 9 Hz, 2H), 7.26 (dd, *J* = 9, 6 Hz, 2H).

### Example 1

### Preparation of (3E)-1-[(1S)-1-(4-fluorophenyl)ethyl]-3-[3-methoxy-4-(4-methyl-1H-imidazol-1-yl)benzylidene]piperidin-2-one

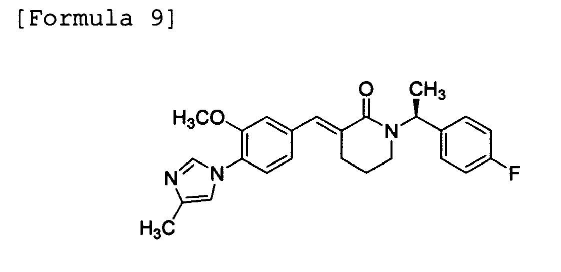

A mixed solution of diisopropylamine (82.6 mL, 588 mmol) in toluene (655 ml) and tetrahydrofuran (45.7 mL) was cooled to -20°C, and a 20% solution of *n-*butyllithium in cyclohexane (172.8 g, 540 mmol) was added dropwise to the mixed solution at the same temperature. The reaction mixture was stirred at -20°C for 76 minutes. The resulting solution was added dropwise to a solution of 1-[1-(4-fluorophenyl)ethyl]piperidin-2-one (124.6 g, 563 mmol) in toluene (507 mL) at -20°C over 76 minutes. The reaction solution was stirred at -20°C for 72 minutes, and then a solution of 3-methoxy-4-(4-methylimidazol-1-yl)benzaldehyde (101.5 g, 469 mmol) in tetrahydrofuran (1217 mL) was added dropwise to the reaction mixture at -20°C over 88 minutes. The reaction mixture was stirred at the same temperature for 72 minutes, and then acetic anhydride (143.7 g, 1.41 mol) was added dropwise to the reaction mixture over 10 minutes. The reaction solution was warmed to 25°C and stirred for about 16 hours. The reaction mixture was cooled to 8°C, and sodium *tert-*butoxide (157.8 g, 1.64 mol) was added thereto in three equal portions at about 30-min intervals. The reaction mixture was stirred at the same temperature for 2 hours, and then water (507 ml) was added dropwise thereto. The reaction mixture was warmed to 26°C, and the resulting organic layer was washed with water (507 mL) to obtain a toluene solution (2882 g) containing 182.2 g (yield: 93%) of the crude title compound.

### Example 2

### Preparation of crystals of (3E)-1-[(1S)-1-(4-fluorophenyl)ethyl]-3-[3-methoxy-4-(4-methyl-1H-imidazol-1-yl)benzylidene]piperidin-2-one

1 N-HC1 (101.5 mL) was added to a portion of the toluene solution in Example 1 which contained the title compound (576.3 g: contain 36.4 g of the title compound) and the aqueous layer was partitioned. 1 N-HCl (60.9 mL) was added to the organic layer, and the aqueous layer was partitioned. The aqueous layers thus obtained were combined. Isopropyl acetate (345 mL), 5 N-NaOH (30.8 mL) and a 5% aqueous sodium hydrogen carbonate solution (13.4 mL) were sequentially added thereto, and the organic layer was partitioned. The resulting organic layer was washed twice with water (101.5 mL × 2) and concentrated under reduced pressure. Isopropyl acetate (182 mL) was added to the concentrated solution, which was further concentrated. Isopropyl acetate was added to the concentrated suspension so as to adjust the total weight to about 323 g. The suspension was then heated at 80°C to dissolve the crystals. The reaction solution was cooled to 60°C, followed by addition of seed crystals. The suspension from which the crystals were precipitated was stirred at an internal temperature of 60°C to 50°C for 1 hour or more, and then gradually cooled to about 8°C over 4 hours. After stirring at the same temperature overnight, the crystals of the title compound was collected by filtration and washed with isopropyl acetate/heptane (91 mL/91 mL). The resulting crystals were dried under reduced pressure at 60°C to obtain 31.1 g (yield: 85%, Purity (by HPLC): 99.7%) of crystals of the title compound.

¹H-NMR (600 MHz, CDCl₃) δ (ppm): 1.55 (d, *J* = 7 Hz, 3H), 1.69 (m, 1H), 1.82 (m, 1H), 2.32 (s, 3H), 2.76 (m, 1H), 2.82 (m, 1H), 2.94 (m, 1H), 3.24 (m, 1H), 3.86 (s, 3H), 6.23 (q, *J* = 7 Hz, 1H), 6.94 (s, 1H), 7.02 (overlapped, 2H), 7.04 (overlapped, 1H), 7.05 (overlapped, 1H), 7.25 (d, *J* = 5 Hz, 1H), 7.32 (dd, *J* = 8, 5 Hz, 2H), 7.76 (brs, 1H), 7.89 (brs, 1H).

### Example 3

### Preparation of crystals of (3E)-1-[(1S)-1-(4-fluorophenyl)ethyl]-3-[3-methoxy-4-(4-methyl-1H-imidazol-1-yl)benzylidenelpiperidin-2-one

(1) 1.5 N-HCl (31.5 mL) was added to a portion of the toluene solution in Example 1 which contained the title compound (285 g: contain 18.0 g of the title compound) and the aqueous layer was partitioned. Water (36 mL) was added to the organic layer, and the aqueous layer was partitioned. The aqueous layers thus obtained were combined. Water (38 mL) and acetone (85 mL) were added to the resulting aqueous solution, and 5 N-NaOH (9.5 mL) was added dropwise thereto at about 21°C (pH 12-12.5). After addition of water (58 mL) to the mixture, stirring the mixture was continued for 2 hours 50 minutes. Then, the oily precipitate was confirmed. Thereafter, when the temperature of the mixture was changed to 8°C, the precipitate was crystallized. After stirring the slurry from which the crystals were precipitated at the same temperature overnight, the crystals of the title compound were collected by filtration and washed with water. The resulting crystals were dried under reduced pressure at 60°C to obtain 16.85 g (yield: 93%, purity (by HPLC): 96.2%) of crude crystals of the title compound. NMR data matched that of the compound of Example 2.

(2) Isopropyl acetate (90 mL) was added to 10.12 g of crude crystals of the title compound (equivalent to 10.0 g of content), and the mixture was warmed to about 80°C, followed by dissolution. The solution was cooled to 63.5°C, and then seed crystals were added thereto. The resulting mixture was gradually cooled to room temperature to promote precipitation of crystals. After stirring the suspension from which the crystals were precipitated at 8°C overnight, the crystals of the title compound were collected by filtration and washed with isopropyl acetate/heptane (1/1). The resulting crystals were dried under reduced pressure at 60°C to obtain 8.24 g (yield: 82%, Purity (by HPLC): 99.6%) of the title compound. NMR data matched that of the compound of Example 2.

### Example 4

### Preparation of (3E)-1-[(1S)-1-(4-fluorophenyl)ethyl]-3-[3-methoxy-4-(4-methyl-1H-imidazol-1-yl)benzylidene]piperidin-2-one

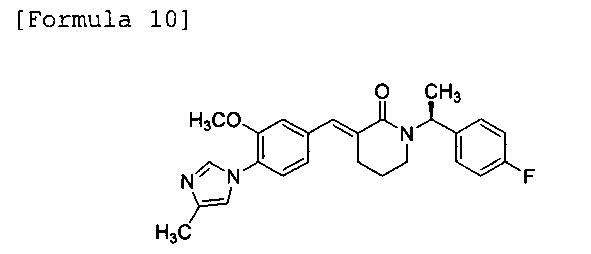

A mixed solution of diisopropylamine (81.9 mL, 583 mmol) in toluene (650 ml) and tetrahydrofuran (47.3 mL) was cooled to -20°C, and a 20% solution of *n-*butyllithium in cyclohexane (179.2 g, 560 mmol) was added dropwise to the mixed solution at the same temperature. The mixture was stirred at -20°C for 82 minutes. The resulting solution was added dropwise to a solution of 1-[1-(4-fluorophenyl)ethyl]piperidin-2-one (113.5 g, 513 mmol) in toluene (504 mL) at -20°C over 47 minutes. The reaction mixture was stirred at - 20°C for 59 minutes, and then a solution of 3-methoxy-4-(4-methylimidazol-1-yl)benzaldehyde (100.8 g, 466 mmol) in tetrahydrofuran (1210 mL) was added dropwise thereto at -20°C over 43 minutes. The reaction mixture was stirred at the same temperature for 57 minutes, and then acetic anhydride (143.0 g, 1.40 mol) was added dropwise thereto over 5 minutes. The reaction solution was warmed to 25°C and stirred for about 16 hours. The reaction mixture was cooled to 8°C, and sodium *tert-*butoxide (156.8 g, 1.63 mol) was added thereto in three equal portions at about 30-min intervals. The reaction mixture was stirred at the same temperature for 28 minutes, and then stirring was continued at 25°C for about 2 hours. Thereafter, water (504 mL) was added dropwise to the reaction mixture. The partitioned organic layer was washed with water (504 mL, in three equal portions) to obtain a toluene solution (2711 g) containing 170.1 g (yield: 87%) of the crude title compound.

### Example 5

### Preparation of crystals of (3E)-1-[(1S)-1-(4-fluorophenyl)ethyl]-3-[3-methoxy-4-(4-methyl-1H-imidazol-1-yl)benzylidene]piperidin-2-one

(1) A portion of the toluene solution in Example 4, which contains the title compound (542 g: contain 34.0 g of the title compound), was concentrated under reduced pressure. The concentrated solution was diluted with toluene (170 ml), and then concentrated under reduced pressure. The same operation was repeated twice. Toluene was added to the concentrated solution so that the amount of toluene was about 88 mL. Thereafter, ethyl acetate (102 mL) was added thereto. The mixture was heated at 50°C for 2 minutes, and then cooled, followed by addition of seed crystals at 40°C. The suspension from which the crystals were precipitated was stirred at 30°C for 1 hour, and heptane (170 ml) was added dropwise thereto over 1 hour 10 minutes. The suspension was gradually cooled to about 8°C, and stirring was further continued at the same temperature overnight. The crystals of the title compound were collected by filtration and washed with ethyl acetate/heptane (1/2, 168 mL). The resulting crystals were dried under reduced pressure at 60°C to obtain 31.1 g (yield: 92%, purity (by HPLC): 99.2%) of crude crystals of the title compound.

(2) Ethyl acetate (210 mL) was added to 30 g of crude crystals of the title compound, and the mixture was warmed to about 60°C, followed by dissolution. The resulting solution was cooled to 50°C, and then seed crystals were added thereto. The suspension from which the crystals were precipitated was stirred at 40°C for 1 hour, and heptane (150 mL) was added dropwise thereto over 1 hour. The resulting suspension was gradually cooled to about 8°C, and stirring was further continued at the same temperature overnight. The crystals of the title compounds were collected by filtration and washed with ethyl acetate/heptane (1/2, 200 mL). The resulting crystals were dried under reduced pressure at 60°C to obtain 24.50 g (yield: 82%, purity (by HPLC): 99.6%) of the title compound.

### Industrial Applicability

In accordance with the present invention, high quality cinnamide derivatives, particularly (3E)-1-[(1*S*)-1-(4-fluorophenyl)ethyl]-3-[3-methoxy-4-(4-methyl-1*H*-imidazol-1-yl)benzylidene]piperidin-2-one, can be prepared in an efficient manner.

## Claims

1. A method for preparing a compound of the following formula (1) or a pharmaceutically acceptable salt thereof: 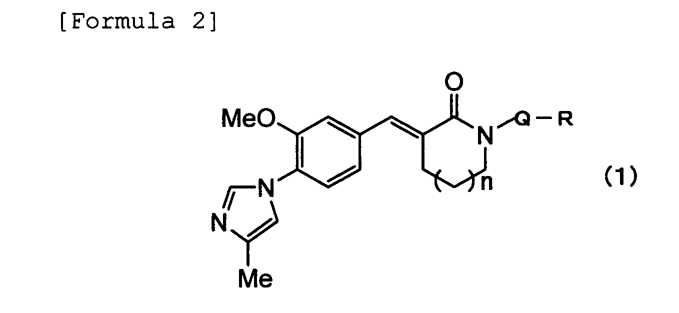 wherein R represents a 6- to 14-membered aromatic hydrocarbon ring group or a 5- to 14-membered aromatic heterocyclic group, both of which may have a substituent; Q represents a single bond or -CH(Y)-wherein Y represents a hydrogen atom or an alkyl group having 1 to 6 carbons; and n represents 0 to 2,
comprises reacting a compound represented by the following formula (2): 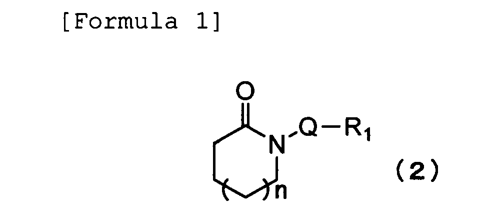 wherein R₁ represents a 6- to 14-membered aromatic hydrocarbon ring group or a 5- to 14-membered aromatic heterocyclic group, both of which may have an optionally protected substituent; and Q and n have the same meanings as described above,
with 3-methoxy-4-(4-methyl-1*H*-imidazol-1-yl)benzaldehyde in the presence of a base and, if necessary, removing a protecting group.

2. The method according to claim 1, wherein the base is a C1-6 alkyllithium or a lithium amide compound.

3. The method according to claims 1 or 2, wherein the product obtained by a reaction using a C1-6 alkyllithium or a lithium amide compound as a base is further subjected, without isolation, to a reaction using an alkali metal C1-6 alkoxide, an amine, an alkali metal hydroxide or a carbonate compound as a base.

4. The method according to any one of claims from 1 to 3, wherein the product obtained by a reaction using a C1-6 alkyllithium or a lithium amide compound as a base is further subjected, without isolation, to a reaction using an alkali metal C1-6 alkoxide or an amine compound.

5. The method according to any one of claims from 1 to 4, wherein Q in the formulas (1) and (2) is - CH(CH₃)-, R in the formula (1) and R₁ in the formula (2) are a 4-fluorophenyl group.
